# EUROPEAN PATENT APPLICATION

(11) **EP 3 971 213 A1**
(43) Date of publication of application: **23.03.2022**
(21) Application number: 20806669.6
(22) Date of filing: 13.05.2020
(51) Int. Cl.: C07K 16/40, G01N 33/573, C12N 9/12

(54) **METHOD FOR PREDICTING THERAPEUTIC RESPONSE TO SERINE-THREONINE KINASE BRAF INHIBITOR DRUGS**

(30) Priority: 13.05.2019 ES 201930419
(71) Applicant: Consejo Superior De Investigaciones Científicas, 28006 Madrid (ES); Universidad de Cantabria, 39005 Santander-Cantabria (ES)
(72) Inventor: CRESPO BARAJA, Pedro, 39011 Santander (Cantabria) (ES); CASAR MARTÍNEZ, Berta, 39011 Santander (Cantabria) (ES)
(74) Representative: Pons
(86) International application number: PCT/ES2020/070308
(87) International publication number: WO 2020/229717

(57) **Abstract**

The present invention relates to an *in vitro* method which enables the prediction of therapeutic response to treatment with BRAF inhibitor drugs, such as vemurafenib, in cancer patients associated with oncogenic mutations in said kinase, such as BRAF positive melanoma. Said method is based on the measurement of cytoplasmic ERK1/2 levels through the detection and quantification of ERK1/2 phosphorylated in serine 301/284, respectively, in an isolated biological sample of the patient. The invention also provides a specific antibody against ERK1/2 phosphorylated in serine 301/284 and a kit comprising it, which are of use in the described method.

LISTADO DE SECUENCIAS

<110>  Consejo Superior de Investigaciones Científicas (CSIC)
       Universidad de Cantabria

<120>  MÉTODO PARA PREDECIR LA RESPUESTA TERAPÉUTICA A FÁRMACOS
       INHIBIDORES DE LA SERINA-TREONINA KINASA BRAF

<130>  PCT1641.1461

<150>  ES P201930419
<151>  2019-05-13

<160>  1

<170>  PatentIn version 3.5

<210>  1
<211>  19
<212>  PRT
<213>  Artificial Sequence

<220>
<223>  Péptido utilizado para la inmunización para generar el anticuerpo
       de la invención

<400>  1

Asn Arg Leu Phe Pro Asn Ala Asp Ser Lys Ala Leu Asp Leu Leu Asp
1               5                   10                  15


Lys Met Leu

## Description

The present invention falls within the field of oncology, specifically within the methods for predicting the response to serine-threonine kinase BRAF inhibitor drugs in cancer patients carrying oncogenic BRAF mutations (BRAF positive). These methods enable the early identification of patients who will benefit from said treatment prior to the administration thereof. In particular, the invention provides a biomarker, as well as a specific antibody against it, useful for predicting which patients will show sensitivity and, therefore, an adequate clinical response to subsequent treatment with BRAF inhibitors.

### BACKGROUND OF THE INVENTION

60% of melanomas carry oncogenic mutations in the serine/threonine kinase BRAF. Currently, the only effective treatment for these cases is by means of administering specific inhibitors of said kinase (for example, vemurafenib and dabrafenib). Although most cases respond to treatment, for reasons still unknown, approximately 30% of patients are resistant to said therapy, so these patients are subjected to a useless treatment, not without adverse side effects. In addition, considering that said treatment costs around €30,000 per patient, these cases represent an unnecessary and completely sterile waste of financial resources for health systems.

The existence of patients that are resistant to treatment with BRAF inhibitor drugs makes it necessary to develop methods of prediction that can be applied in clinical practice to identify these patients at an early stage. This approach would help to design effective treatment strategies tailored to each patient.

The characterisation of molecular markers that make it possible to discriminate between cases that can respond to treatment with BRAF inhibitors and those that do not, among all BRAF positive melanoma patients, is of crucial importance. Being able to determine early whether the patient will have a favourable response to treatment before administering the same would be very useful in clinical practice to be able to select the appropriate treatment for each individual case. This in turn would make it possible to improve the prognosis of the subject.

However, no biomarker or methodology is currently available to predict, reliably and simply, the clinical response to treatment with BRAF inhibitor drugs in these patients before proceeding with the administration thereof. In other words, there are no methods that can be applied in clinical practice which make it possible to identify non-responding patients, which prevents predicting the efficacy of the therapy.

A few years ago it was described that the response to vemurafenib is positively correlated with the cytoplasmic levels of MAP kinase ERK1/2 (Bollag G et al., 2010, Nature, 467: 596-599). In this regard, it is also worth mentioning that cytoplasmic ERK1/2 levels correlate with a good prognosis in invasive breast tumours and in small cell lung tumours (Blackhall FH. et al., 2007, Clin Cancer Res, 9: 2241-2247; Nakopoulou L. et al., 2007, APMIS, 113: 693-701). Unfortunately, to date it has not been possible to apply this concept to the clinic, since there is no easy way to discriminate between cytoplasmic ERK1/2 and nuclear ERK1/2, except for cumbersome microscopic techniques (Bollag G etal., 2010, Nature, 467: 596-599), impractical for routine prognosis.

Therefore, it is necessary to have adequate tools that can be used in clinical practice which enable the selective identification of cytoplasmic ERK1/2 versus nuclear ERK1/2, since these tools would make it possible to predict the response of patients with BRAF positive cancer, preferably melanoma, to the therapeutic administration of BRAF inhibitor drugs, such as vemurafenib.

### DESCRIPTION OF THE INVENTION

The present invention solves the problem raised above by means of a method that makes it possible to discriminate between cytoplasmic ERK1/2 and nuclear ERK1/2. This method uses, in particular, the detection and quantification, in isolated samples from BRAF positive cancer patients, of ERK1/2 phosphorylated in serine 301/284 respectively, which have been shown in the present invention to be present only in the cytoplasmic fraction of ERK. The quantification of the levels of ERK1/2 phosphorylated in serine 301/284 thus makes it possible to predict the clinical response to subsequent treatment with BRAF inhibitors.

The inventors of the present invention have identified a new phosphorylation site in ERK2, specifically in serine 284 (serine 301 in ERK1) and, after generating an antibody that specifically recognises ERK1 and ERK2 phosphorylated in serine 301/284 respectively, they have verified that said phosphorylation occurs, only and exclusively, in the cytoplasmic fraction of ERK1/2. Thus, using protein electrophoresis techniques, in the present invention it is demonstrated that the levels of ERK1/2 phosphorylated in serine 301/284 are much higher in melanoma cell lines that respond adequately to treatment with the BRAF inhibitor drug vemurafenib, in comparison with the levels found in lines resistant to said drug. Therefore, the quantification of the levels of ERK1/2 phosphorylated in serine 301/284 in isolated biological samples of cancer patients carrying oncogenic BRAF mutations (BRAF positive) makes it possible to predict the therapeutic response to the administration of BRAF inhibitors.

Likewise, the anti-ERK1/2 phosphorylated in serine 301/284 antibody generated in the present invention can be used in the method for predicting the therapeutic response to BRAF inhibitors proposed herein for the quick and easy stratification of BRAF positive cancer cases, preferably melanoma, based on the potential thereof for response to BRAF inhibitor therapy.

Given the foregoing, one aspect of the invention relates to the use of the levels of ERK1 phosphorylated in serine 301 and of the levels of ERK2 phosphorylated in serine 284 as a biomarker to predict *in vitro* the therapeutic response to treatment with a serine-threonine kinase BRAF inhibitor drug in a patient.

"To predict the therapeutic response" refers to determining, before administering the treatment, whether the BRAF inhibitor drug will induce a favourable, positive or adequate response in the subject or patient once treated with the same, in other words, after administering the drug. A "positive or adequate response" to a BRAF inhibitor drug occurs when there is an improvement or reduction in symptoms of the tumour, preferably cancer, more preferably melanoma, in the patient. Said positive or adequate response to treatment with a BRAF inhibitor occurs, in particular, when the total activity levels of ERK1/2, assessed by means of the phosphorylation levels of the TEY phosphorylation motif thereof, are decreased in an isolated biological sample of the patient after drug administration compared to said activity levels measured in an isolated biological sample of the same patient prior to drug administration. Said positive or adequate response to treatment with a BRAF inhibitor also occurs, preferably, when there is disappearance, or decrease, of macroscopic metastatic lesions.

The term *"in vitro"* means that the methods and uses described in the invention are carried out entirely outside the human or animal body.

The term "subject", "individual" or "patient", as used in the present invention, preferably refers to a human or non-human mammal, such as primates, equines, rodents, ruminants, cats or dogs. Preferably, the patient to whom the invention refers is a human being.

"ERK1/2" refers to, preferably, human ERK1 (MAPK1) and ERK2 (MAPK3) (MAPK1 (UniprotKB: P28482) and MAPK3 (UniprotKB: P27361) according to the HUGO nomenclature). They are MAP kinases involved in various cellular functions, such as regulation of meiosis, mitosis and postmitosis in differentiated cells. A variety of stimuli, including growth factors, cytokines, viral infection, carcinogenic agents, etc., activate the RAS-ERK signalling pathway. ERK1/2 are rapidly phosphorylated upon activation of cell surface receptor tyrosine kinases, such as the epidermal growth factor receptor. This phosphorylation leads to the activation of the kinase activity thereof.

The "ERK1/2 phosphorylated in serine 301 or in serine 284, respectively" are the ERK1 and ERK2 enzymes that have incorporated a phosphate group at said specific positions in the amino acid sequence thereof.

The enzyme "serine-threonine kinase BRAF" is the enzyme, preferably human, (UniprotKB: P15056), more preferably encoded by the gene 7q34. The term "BRAF positive" refers to, in the present invention, the presence of a substitution (oncogenic) mutation at residue V600 of the amino acid sequence of BRAF, preferably the presence of the substitution V600E.

In another preferred embodiment, the serine-threonine kinase BRAF inhibitor is vemurafenib and/or dabrafenib, more preferably vemurafenib.

The drug "vemurafenib" (PLX4032 or RG7204) is a low molecular weight chemical compound that is administered orally, inhibitor of the activity of the serine-threonine kinase BRAF enzyme. The therapeutic indication for this BRAF inhibitor is in BRAF V600 mutation-positive unresectable or metastatic melanoma. It is marketed under the name Zelboraf^{®}.

The drug "dabrafenib" is also a BRAF inhibitor and its therapeutic indication is in BRAF V600 mutation-positive unresectable or metastatic melanoma, as monotherapy treatment or in combination with trametinib, and in BRAF V600 mutation-positive advanced non-small cell lung cancer, in combination with trametinib. It is marketed under the name Tafinlar^{®}.

Oncogenic mutations in the encoding gene for the BRAF enzyme, which lead to the substitution of the amino acid Valine (V) at position 600, preferably by the aspartic amino acid (E), lead to constitutive activation of this protein, which promotes cell proliferation in the absence of growth factors that are normally required for said proliferation. Oncogenic BRAF mutations have been identified very frequently in specific types of cancers, being present, for example, in 60% of melanomas. The most frequently observed oncogenic BRAF mutation is V600E, which represents approximately 90% of the BRAF mutations observed in cases of melanoma.

To that end, in another preferred embodiment, the patient referred to in the present invention suffers from cancer, in particular BRAF positive cancer, in other words, he or she is a cancer patient presenting an oncogenic BRAF mutation wherein said mutation is preferably V600, more preferably the mutation is V600E.

More preferably, the cancer is melanoma, even more preferably BRAF V600 mutation-positive unresectable or metastatic melanoma, or non-small cell lung cancer, even more preferably BRAF V600 mutation-positive advanced non-small cell lung cancer. In a particular embodiment, the cancer is melanoma.

In the most preferred embodiment of the present invention, the BRAF inhibitor is vemurafenib and the patient suffers from melanoma.

As cytoplasmic ERK1/2 levels are correlated, not only with the therapeutic response to treatment with a BRAF inhibitor drug, but also with a good prognosis in breast tumours, preferably invasive tumours, and in lung tumours, preferably small cell tumours (Blackhall FH. et al., 2007, Clin Cancer Res, 9: 2241-2247; Nakopoulou L. et al., 2007, APMIS, 113: 693-701), another aspect of the invention relates to the use of the levels of ERK1 phosphorylated in serine 301 and of the levels of ERK2 phosphorylated in serine 284 as a biomarker for the *in vitro* prognosis of breast tumours, preferably invasive tumours, and/or lung tumours, preferably small cell tumours, in a patient.

The term "prognosis" refers to the method by means of which a prediction of the events that will occur in the development or course of a disease, preferably, cancer, is established, including relapse or capacity for metastatic spread.

Another aspect of the invention relates to an *in vitro* method, hereinafter "method of the invention", to predict the therapeutic response to treatment with a serine-threonine kinase BRAF inhibitor drug in a patient, wherein said method comprises the following steps:
a. Quantifying the levels of ERK1 phosphorylated in serine 301 and of ERK2 phosphorylated in serine 284 in an isolated biological sample of the patient (collected prior to administering the treatment with the BRAF inhibitor),
b. Comparing the levels quantified in step (a) with a reference value, wherein said reference value comes from the quantification of the levels of ERK1 phosphorylated in serine 301 and of ERK2 phosphorylated in serine 284 in an isolated biological sample of a patient who does not respond to treatment with a serine-threonine kinase BRAF inhibitor, and
c. Assigning the patient from step (a) to the group of individuals who will respond adequately to treatment when the quantification value obtained in step (a) is significantly higher than the reference value.

The term "quantifying" refers to the measurement of the amount or concentration of ERK1 phosphorylated in serine 301 and of ERK2 phosphorylated in serine 284 in the sample.

The term "amount" refers to, but is not limited to, the absolute or relative amount of ERK1 phosphorylated in serine 301 and of ERK2 phosphorylated in serine 284 in the sample, as well as any other value or parameter related thereto or that can be derived from it. These other values or parameters comprise, for example, intensity values of a signal obtained from any physical or chemical property of ERK1 phosphorylated in serine 301 and of ERK2 phosphorylated in serine 284 obtained by means of indirect or direct measurements, for example, by means of mass spectroscopy or by means of nuclear magnetic resonance.

Thus, the quantification of the levels of ERK1 phosphorylated in serine 301 and of ERK2 phosphorylated in serine 284, as described in the present invention, can be done as a direct or indirect measurement. Direct measurement refers to the measurement of the intensity of a signal directly obtained from the presence of ERK1 phosphorylated in serine 301 and of ERK2 phosphorylated in serine 284. This signal is directly correlated with the number of product molecules present in the sample. This signal, also called "intensity signal", can be obtained, for example, by measuring an intensity value derived from a physical or chemical property of the product. Indirect measurement refers to the measurement obtained from a secondary component (i.e., a component that is different from ERK1 phosphorylated in serine 301 and ERK2 phosphorylated in serine 284) or a measurement derived from, for example, cellular responses, ligands, substrates, labels or enzyme reaction products associated with ERK1 phosphorylated in serine 301 and ERK2 phosphorylated in serine 284 or the activities thereof.

Methods for detecting and quantifying levels of ERK1 phosphorylated in serine 301 and levels of ERK2 phosphorylated in serine 284 are known to those skilled in the art, for example, by means of large-scale phosphoproteomics studies using the mass spectrometry technique.

This quantification of the levels of ERK1 phosphorylated in serine 301 and the levels of ERK2 phosphorylated in serine 284 can be performed, but is not limited to, by means of incubation or hybridisation *in situ with* specific antibodies against (which recognise) ERK1 phosphorylated in serine 301 and/or ERK2 phosphorylated in serine 284, or an immunologically active fragment thereof, in assays such as Western blot, electrophoresis gels, membrane transfer and hybridisation with specific probes, immunoprecipitation assays, protein arrays, preferably antibody-based microarrays, flow cytometry, immunofluorescence, immunohistochemistry, chemiluminescence, immunoassays such as ELISA, radioimmunoassay (RIA), or any other enzymatic method, by means of incubation with a specific ligand or substrate, lateral flow devices or Luminex^{®}, NMR or any other diagnostic imaging technique using paramagnetic nanoparticles or other types of functionalised nanoparticles, by means of chromatographic techniques preferably combined with mass spectrometry, colorimetry or isoelectric focusing. Measurement of the levels of ERK1 phosphorylated in serine 301 and/or ERK2 phosphorylated in serine 284 can be carried out by the specific recognition of any fragment of the enzymes thus phosphorylated by means of probes and/or antibodies.

Preferably, the quantification of the levels of ERK1 phosphorylated in serine 301 and/or ERK2 phosphorylated in serine 284 is carried out in the present invention by means of immunoassay based on specific antibodies against said forms of ERK, more preferably by means of Western blot. For the immunoassay, the antibodies used may be marked or unmarked, for example, they can be marked with a secondary antibody, an enzyme, a substrate of an enzyme, radioisotopes, magnetic labels, fluorescence or the like, and/or they can be immobilised on a support such as a microtiter plate or bio-chip.

An "immunoassay" or "immunohistochemical assay" is a biochemical assay that detects and/or quantifies the amount or concentration of one or more peptides of interest (in the context of the present invention ERK1 phosphorylated in serine 301 and ERK2 phosphorylated in serine 284), in a sample. To that end, the reaction uses one or more antibodies specific to the antigens to be detected. Protein quantification can be carried out by means of any method known in the art, for which the antigen and/or the antibody will preferably be marked. The immunoassay to which the present invention relates can be competitive or non-competitive. In a competitive immunoassay, the detected signal will be inversely proportional to the concentration of antigen in the sample. In a non-competitive immunoassay (such as a sandwich ELISA), the detected signal is directly proportional to the concentration of antigen in the sample.

Examples of useful immunoassays to be applied in the method of the present invention are, but not limited to, immunoblotting (Western blot), immunoprecipitation, ELISA, multiplex assay, dipstick assay, line immunoassay (LIA), radioimmunoassay (RIA), immunoradiometric assay (IRMA), immunofluorescence, immunohistochemistry, chemiluminescence, passive haemagglutination, immunolabelling with gold particles (transmission electron microscopy), lipopolysaccharide (LPS) chips or protein or x-map, immunoquantitative real-time PCR (iqPCR), electrochemiluminescent labels, label-free immunoassays (for example, surface plasmon resonance), photoacoustic immunoassay, cloned enzyme donor immunoassay (CEDIA), lateral flow immunochromatographic assays, magnetic immunoassay (MIA), surround optical-fibre immunoassay (SOFIA), CD/DVD-based immunoassay or agglutination-PCR (ADAP).

The ELISA assay is based on the immobilisation of an antigen or antibody on a solid support, so that this system is subsequently put in contact with a fluid phase that contains the complementary reagent, which can be bound to a label or marker compound. The ELISA technique referred to in the present invention can be direct, indirect or sandwich.

The antibody used in the methods described herein is preferably conjugated with a detection system or is bound to a secondary antibody which is coupled to a detection system. The signal produced as a consequence of the marking reaction can be measured, for example, but not limited to, by means of spectrophotometry, chemiluminescence, spectrofluorometry, bioluminescence, differential calorimetry, analytical ultracentrifugation, interferometry, etc. Preferably, the technique used in the present invention is chemiluminescence.

The expression "collected before administering the treatment with the BRAF inhibitor" refers to a biological sample collected from the patient when said patient has not yet received prior treatment with BRAF inhibitors, preferably with vemurafenib.

The term "isolated biological sample" refers to, but is not limited to, any tissue and/or biological fluid obtained or extracted from a subject or patient comprising tumour cells, in other words, cells from a tumour. The biological sample can be, for example, a tissue from a tumour biopsy or a sample from fine needle aspiration, or it can be a biological fluid, for example, blood, plasma, serum, lymph, ascites fluid, urine, saliva or glandular exudate. The biological sample can be fresh, frozen, fixed, or fixed and embedded in paraffin.

The term "reference value", as used in the method of the invention described above, is any value or range of values derived from the quantification of the levels of ERK1 phosphorylated in serine 301 and of ERK2 phosphorylated in serine 284 in an isolated biological sample of a patient known not to respond adequately to treatment with a BRAF inhibitor, preferably vemurafenib, or in a mixture of isolated biological samples from patients of this type.

The "comparison" referred to in step (b) of the method of the invention can be carried out manually or in an automated manner.

An amount "significantly greater" than a reference value can be determined using various statistical tools, such as, for example, but not limited to, determination of confidence intervals, determination of the p-value, Student's T test, Fisher's discriminant functions, Kruskal-Wallis, ANOVA, Bonferroni, Mann-Whitney, etc.

The steps (a) and (b) of the method of the invention can be totally or partially computerised. In addition, the method of the invention may comprise other additional, optional steps, for example, related to the pre-treatment of biological samples before the analysis thereof.

In another preferred embodiment of the method of the invention, the serine-threonine kinase BRAF inhibitor is vemurafenib and/or dabrafenib, more preferably vemurafenib.

In another preferred embodiment, the patient to whom the method of the invention is to be applied suffers from cancer, in particular BRAF positive cancer, in other words, he or she is a cancer patient presenting an oncogenic BRAF mutation wherein said mutation is preferably V600, more preferably the mutation is V600E.

More preferably, the cancer is melanoma, even more preferably BRAF V600 mutation-positive unresectable or metastatic melanoma, or non-small cell lung cancer, even more preferably BRAF V600 mutation-positive advanced non-small cell lung cancer. In a particular embodiment, the cancer is melanoma.

In a particular embodiment of the method of the invention, the serine-threonine kinase BRAF inhibitor is vemurafenib and the patient suffers from melanoma.

In another preferred embodiment, the patient to whom the method of the invention is to be applied is human.

In another preferred embodiment, the quantification of the levels of ERK1 phosphorylated in serine 301 and of ERK2 phosphorylated in serine 284 is carried out, in the methods described herein, by using an antibody that recognises ERK1 phosphorylated in serine 301 and ERK2 phosphorylated in serine 284 specific against the peptide consisting of SEQ ID NO: 1 (NRLFPNADSKALDLLDKML), in other words, by means of the antibody of the invention described below.

Another aspect of the invention relates to an *in vitro* method for the prognosis of breast tumours, preferably invasive tumours, and/or lung tumours, preferably small cell tumours, in a patient, wherein said method comprises the following steps:
a. Quantifying the levels of ERK1 phosphorylated in serine 301 and of ERK2 phosphorylated in serine 284 in an isolated biological sample of the patient,
b. Comparing the levels quantified in step (a) with a reference value, wherein said reference value comes from the quantification of the levels of ERK1 phosphorylated in serine 301 and of ERK2 phosphorylated in serine 284 in an isolated biological sample of a patient diagnosed with the same type of cancer as the patient in step (a) and who has a bad prognosis, and
c. Assigning the patient from step (a) to the group of individuals who will have a good prognosis when the quantification value obtained in step (a) is significantly higher than the reference value from step (b).

"Bad prognosis" is understood to mean the relapse, metastasis and/or absence of improvement in symptoms of the tumour, preferably cancer.

The antibody described in the present invention, which recognises ERK1 phosphorylated in serine 301 and ERK2 phosphorylated in serine 284 and that is specific against the peptide of SEQ ID NO: 1, has been designed by the inventors of the present invention to develop the methods described herein. Therefore, another aspect of the invention relates to an antibody, which recognises ERK1 phosphorylated in serine 301 and ERK2 phosphorylated in serine 284, specific against the peptide consisting of SEQ ID NO: 1 (NRLFPNADSKALDLLDKML). Hereinafter this antibody will be referred to as "antibody of the invention".

This antibody of the invention is polyclonal, in other words, it has been generated by immunising an animal, preferably a non-human mammal, more preferably a rabbit, with the peptide (antigen) designed by the inventors consisting of SEQ ID NO: 1. Methods of production and purification of polyclonal antibodies by immunising an animal with the corresponding antigen are widely known to those skilled in the art, and any of them could be used to obtain the antibody of the invention.

In the peptide consisting of SEQ ID NO: 1, the serine at position 9 is phosphorylated, preferably said serine has been chemically phosphorylated. Said peptide is 100% homologous or identical between the ERK1 and ERK2 paralogs, therefore, the antibody of the invention generated against the same recognises (binds, hybridises to) both proteins phosphorylated in serine 301 and 284 respectively.

Another aspect of the invention relates to a peptide consisting of SEQ ID NO: 1 (NRLFPNADSKALDLLDKML), wherein the serine at position 9 is phosphorylated. Another aspect of the invention relates to the use of said peptide for the generation of the antibody of the invention.

Preferably, the antibody of the invention is marked. The term "marked", as used in the present description, refers to the antibody being conjugated to a label or detection system. A large number of labels that can be conjugated to an antibody are known in the state of the art. Examples of said labels are, but not limited to, radioisotopes [for example, 32P, 35S or 3H], fluorescent or luminescent markers [for example, fluorescein (FITC), rhodamine, Texas Red, GFP, phycoerythrin (PE), allophycocyanin, 6-carboxyfluorescein (6-FAM), 2',7'-dimethoxy-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA)]; secondary antibodies or antibody fragments [for example, fragments F(ab)2)], affinity labels [for example, biotin, avidin, agarose, bone morphogenetic protein (BMP), haptens], enzymes or substrates of enzymes [for example, alkaline phosphatase (AP) and horseradish peroxidase (HRP)].

The advantage of marking antibodies is that they can be detected and the signal thereof quantified.

Preferably, the antibody of the invention is immobilised on a support. The term "immobilised", as used in the present description, refers to the fact that the antibody is bound to a support without losing its immunoreaction activity with the antigen thereof (peptide of SEQ ID NO: 1). Preferably, the support is the surface of a matrix (for example, a nylon or latex matrix), a membrane, a microtiter plate (for example, a 96-well plate) or plastic, silicone, glass or similar support, or beads (for example spheres, preferably agarose spheres or small superparamagnetic microspheres made up of biodegradable matrices), gel, cellulosic support, adsorption resin or the like. In general, any solid surface that enables binding, directly or indirectly, by means of covalent or non-covalent bond, of the antibody of the invention can be used. In addition, the support can be in the form of a rod, test strip, paper, latex bead, microsphere, array, multi-well plate or the like.

More preferably, the antibody of the invention is marked and immobilised. Even more preferably, said marking occurs through a horseradish peroxidase-conjugated secondary antibody.

The antibody described in the present invention can be human, humanised, recombinant, monoclonal, chimerical, conjugated, etc. Immunologically active fragments of the antibody of the invention are also included within the scope of the present invention. Examples of said fragments are, but not limited to, a Fab fragment, F(ab')2, Fab', F sc or Fv.

Another aspect of the invention relates to a kit, useful to be used in the methods described herein, hereinafter "kit of the invention", comprising the antibody of the invention, wherein said antibody may or may not be marked and/or immobilised as described above.

This kit comprises, preferably, all those elements necessary to predict the therapeutic response to treatment with a BRAF inhibitor drug in a patient or to predict (the evolution of) breast tumours, preferably invasive tumours, and/or lung tumours, preferably small cell tumours, in a patient, according to the methods described above. Thus, the kit of the invention may comprise elements such as, for example, but not limited to, conservation solutions, buffers, diluents, filters, carriers, enzymes, such as polymerases, cofactors necessary to obtain optimal activity of said enzymes, etc. The kit may comprise all those supports and containers necessary for implementing the methods described herein. The kit may further comprise other molecules, genes, proteins or probes, suitable as positive or negative controls. Preferably, the kit of the invention further comprises instructions on how to carry out the methods described herein, labels to identify the different elements comprised in the kit and their application and/or lists of the components comprised in the kit.

The kit of the invention may comprise, in different combinations, primers or oligonucleotides and control probes, secondary antibodies that are used for marking through the conjugation thereof with a detection system and/or control antibodies that are used for the normalisation of expression levels, reagents, detection signals, instruments necessary for the processing and starting up of arrays, fluorochromes, marking probes, substrates necessary for the activation or initiation of the marking reaction by the selected conjugation system, blocking, stopping and/or washing solutions, or the like. The probes, primers and/or control antibodies can be specific for genes, proteins or peptides constitutively expressed by cells in the biological sample analysed, so that the application thereof makes it possible to ensure that the values of the expression levels of ERK1 phosphorylated in serine 301 and of ERK2 phosphorylated in serine 284 measured in the sample are reliable and correct.

The kit may also comprise suitable means for accommodating and storing the biological sample to be analysed in the methods described herein. Said means can be, for example, a container suitable for holding a sample from a tumour biopsy or aspiration. Thus, said kit may comprise a container such as bottles, vials, syringes or test tubes. Said container can be made of, for example, but not limited to, glass or plastic or any other suitable material for storing the biological sample in optimal conditions.

Another aspect of the invention relates to the use of the antibody of the invention or the kit of the invention for the *in vitro* prediction of the therapeutic response to treatment with a serine-threonine kinase BRAF inhibitor drug in a patient, or for the *in vitro* prognosis (evolution) of breast tumours, preferably invasive tumours, and/or lung tumours, preferably small cell tumours, in a patient, more preferably by means of the methods described above.

In a preferred embodiment of said use, the serine-threonine kinase BRAF inhibitor is vemurafenib and/or dabrafenib, preferably vemurafenib.

In another preferred embodiment, the patient suffers from cancer, in particular BRAF positive cancer, in other words, he or she is a cancer patient with an oncogenic BRAF mutation, wherein said mutation is preferably V600, more preferably the mutation is V600E.

More preferably, the cancer is melanoma, even more preferably BRAF V600 mutation-positive unresectable or metastatic melanoma, or non-small cell lung cancer, even more preferably BRAF V600 mutation-positive advanced non-small cell lung cancer. In a particular embodiment, the cancer is melanoma.

In the most preferred embodiment, the serine-threonine kinase BRAF inhibitor is vemurafenib and the patient suffers from melanoma.

In another preferred embodiment, the patient is human.

Another aspect of the invention relates to an *in vitro* method to obtain information or data that are useful to predict the therapeutic response to treatment with a serine-threonine kinase BRAF inhibitor drug in a patient, wherein said method comprises the following steps:
a. Quantifying the levels of ERK1 phosphorylated in serine 301 and of ERK2 phosphorylated in serine 284 in an isolated biological sample of the patient (collected prior to administering the treatment with the BRAF inhibitor), and
b. Comparing the levels quantified in step (a) with a reference value, wherein said reference value comes from the quantification of the levels of ERK1 phosphorylated in serine 301 and of ERK2 phosphorylated in serine 284 in an isolated biological sample of a patient who does not respond to treatment with a serine-threonine kinase BRAF inhibitor.

These steps (a) and (b) can be carried out as explained above for the method of the invention.

Another aspect of the present invention relates to a method for treating a patient suffering from BRAF positive cancer, preferably BRAF positive melanoma, comprising: (a) identifying the patient as responding or non-responding to treatment with BRAF inhibitors, preferably to treatment with vemurafenib, according to the method of the invention; and (b) administering said treatment to the patient when in step (a) it has been determined that he or she is a responding patient thereto.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and they are not intended to be limiting of the scope of protection of the present invention.

### DESCRIPTION OF THE FIGURES

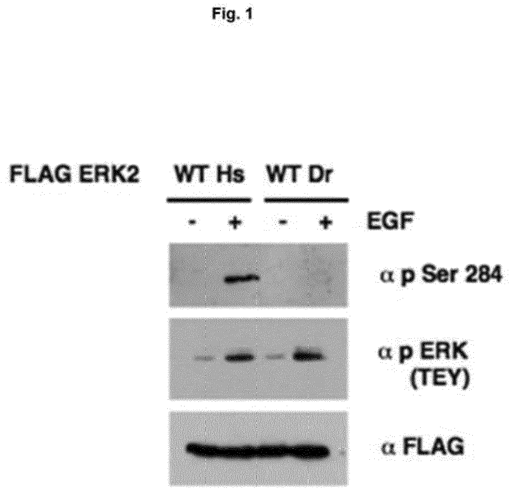

**Fig. 1****. Specificity of the antibody of the invention (anti-phospho ser284/301 antibody).** HEK294 cells were transfected with human (Hs) or zebrafish (Dr) ERK2, wherein the homologous residue of serine 284 is a proline, therefore, not phosphorylatable. It is observed that the antibody only recognises human ERK2, in cells under stimulation, in this case with EGF. An anti-FLAG antibody was used as a control.

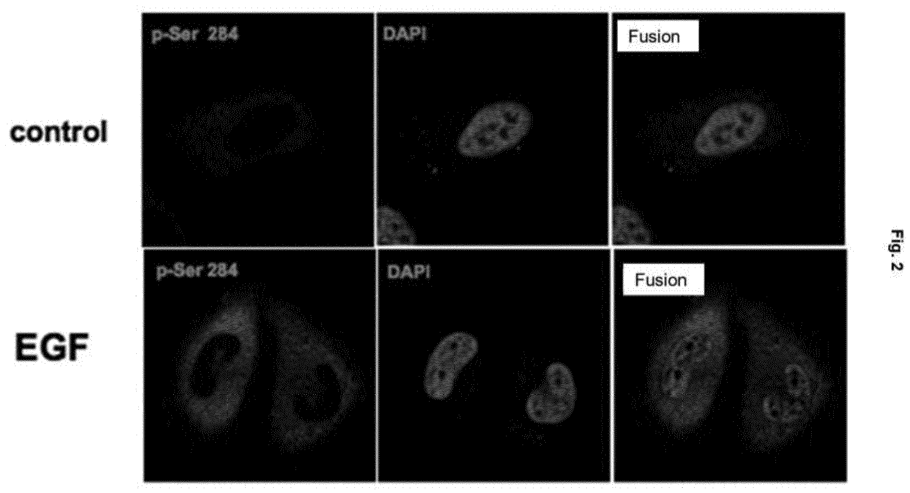

**Fig. 2****. ERK1/2 phosphorylated in ser 301/284 are located exclusively in the cytoplasm.** HeLa cells were stimulated with EGF for 5 min, and the subcellular localisation of phosphorylated endogenous ERK1/2 in the aforementioned residues was analysed by means of immunofluorescence and confocal microscopy. It is observed that ERK1/2 phosphorylated in said positions are completely excluded from the nucleus, marked with DAPI.

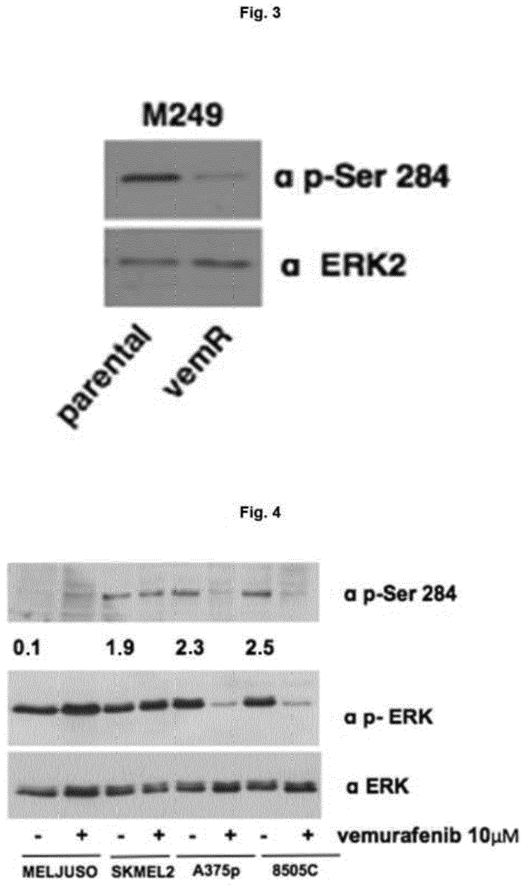

**Fig. 3****. Correlation between the levels of ERK1/2 phosphorylated in ser 301/284 and sensitivity to vemurafenib.** From the BRAF positive melanoma cell line M249, a subline resistant to vemurafenib (vemR) was obtained and the levels of phospho-ser 284/301 were analysed in said subline in comparison with the parental line. It is observed that these levels are much higher in the parental line, sensitive to vemurafenib.
**Fig. 4****. Correlation between the levels of ERK1/2 phosphorylated in ser 301/284 and sensitivity to vemurafenib.** The sensitivity to vemurafenib was evaluated in different lines of melanoma, analysing the reduction in the total activity of ERK1/2, measured by the decrease in the levels of canonical phosphorylation (p-ERK). It is observed that the most sensitive lines are 8505C and A375P. The levels of phospho-ser 284/301 were also analysed in said lines. It is observed that said levels, before treatment with vemurafenib, are much higher than those observed in the resistant lines MELJUSO and SKMEL2.

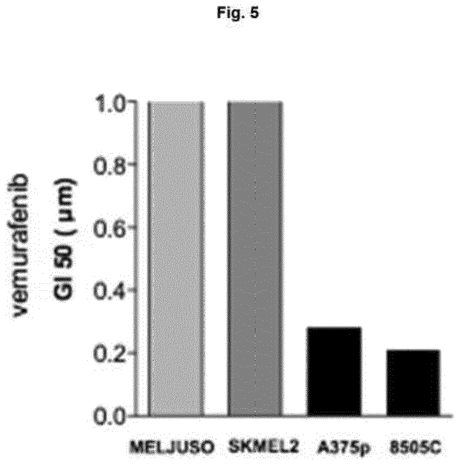

**Fig. 5****. Sensitivity to vemurafenib of the lines of melanoma used in** **Fig.** 4, **evaluated by the concentration necessary to stop proliferation (GI50).** It is observed that the most sensitive lines are 8505C and A375P, which show the highest levels of phospho-ser 284/301 in FIG. 4.

### EXAMPLES

Next, the invention will be illustrated by means of assays carried out by the inventors that show the effectiveness of the method of the invention and of the antibody generated to be used in said method.

### Example 1. Material and methods.

### The cell lines used in the assays were:

HEK293T: Human kidney embryonic cells, T antigen positive.

HeLa: cervical cancer epithelial cells.

A375p: melanoma epithelial cells, having a BRAF mutation.

SKMEL2: melanoma epithelial cells, having a BRAF mutation.

Parental M249 and Vemurafenib-resistant M249: melanoma epithelial cells, having a BRAF mutation.

Mel JUSO: melanoma epithelial cells.

The cell lines were grown in DMEM culture medium supplemented with 10% Foetal Bovine Serum and antibiotics (Penicillin and Streptomycin).

### Western blot:

To obtain the total protein extracts, the cells were lysed with the suitable volume of lysis buffer (20 mM HEPES pH 7.5, 10 Mm EGTA, 40 mM β-glycerophosphate, 1% non-ionic detergent NP40, 2.5 mM MgCl₂, 1mM orthovanadate, 1mM DTT and extemporaneously 10µg/ml aprotinin and 10µg/ml leupeptin). The cells were harvested and centrifuged at 12,000 rpm, for 10 minutes and at 4 °C. The protein extracts were separated from the rest of the components of the cells and the protein concentration of each lysate was quantified. To determine the amount of protein, the Bradford method was used. Approximately 50 µg of protein were taken, to which 5X Laemmli buffer (100 mM Tris pH 6.8, 4% SDS, 20 % glycerol, 20 mM DTT and 0.005% bromophenol blue) was added. After boiling the samples for five minutes, they were subjected to vertical sodium dodecyl sulphate-polyacrylamide gel electrophoresis (SDS-PAGE) of 12% acrylamide. The proteins were transferred to a nitrocellulose membrane setting the amperage at 400 mA for 1 hour. After the transfer had finished, the membranes were incubated for one hour, at room temperature and with stirring, in a solution of TBS-T with 4% BSA, to block unspecific sites. After that, the filters were incubated with the phosphospecific primary antibody of the invention (0.2-0.4 µg/ml diluted in 4% BSA in TBS-T) for one hour at room temperature or overnight at 4 °C while stirring. Two washes with TBS-T were performed for a total of fifteen minutes, after which the filters were incubated with the corresponding peroxidase-conjugated secondary antibody, diluted 1:5,000 or 1:10,000 in 0.4% BSA in TBS-T for one hour at room temperature. Two washes with TBS-T were performed again and the protein was detected by chemiluminescence using the ECL kit. An autoradiography of the filters was carried out with Konica films, which enabled the detection of a band where the primary antibody had specifically recognised the phosphorylated protein of interest. As an internal loading control, a specific antibody that recognises the total protein of interest was used.

### Immunofluorescence

Cells were grown in DMEM 10% Foetal Bovine Serum to subconfluence on sterile 10 mm diameter glass cover slides. At the time of immunofluorescence, cells were washed with 1X PBS and fixed with a 4% solution of paraformaldehyde in 1X PBS, for 10 minutes at room temperature. The cells were permeabilised by incubating for 5 minutes with a 0.5% dilution of Triton X-100 in PBS, followed by three washes of 1X PBS of five minutes each. Next, cells were incubated with the phosphospecific primary antibody of the invention at a concentration of 1/100 for one hour in a humid chamber. After three washes with PBS, of five minutes each, the FITC fluorophore-conjugated secondary antibody was added and incubated for 45-50 minutes in a humid chamber and in the dark. After that time, two new washes were carried out, of five minutes each, con 1X PBS. Lastly, a drop of Prolong mounting medium with DAPI was added on a slide and the cover was then placed on top with the cells facing down. The cells were examined by means of confocal microscopy (Leica TCS SP8). The images were digitised and processed using the Fiji Image J program.

### Immunohistochemistry

Tissue samples or cell pellets were fixed with 4% parapholmaldehyde and embedded in paraffin. The sections were mounted on positively charged slides (Genex-brand^{®}), recommended for immunohistochemistry. Dewaxing was achieved by passing the sections through xylene (10 min), and decreasing graduations of ethyl alcohol (100° 10 min, 96° 5 min, and 70° 5 min). Endogenous peroxidase activity was blocked by incubating sections in 3% hydrogen peroxide solution in methanol for 15 min, and incubation in distilled water for 10 min. Subsequently, the sections were incubated with a 1% BSA bovine serum albumin solution in TBST buffer for 30 min with the intention of blocking non-specific binding sites. Subsequently, sections were incubated with the phosphospecific antibody of the invention at a 1:100 dilution in PBS, overnight at 4 °C, in a humid chamber. The development of the reaction was carried out by the DAKO chromogen DAB technique. Contrast staining was performed by immersing the sections in Mayer's hematoxylin for 1 min; they were then placed under a stream of running water for development. Mounting was done with aqueous mounting medium (VectaMount AQ, Vector Lab Ind). Observation of the preparations was made on a Nikon microscope. Photographs were taken with an Olympus C4000 digital camera.

### Example 2. Results.

To obtain an antibody that specifically recognises the phosphorylated ser 301/284 of ERK1/2, respectively, rabbits were immunised with the peptide consisting of SEQ ID NO: 1 previously mentioned, following the usual protocols routinely used for this purpose. The immunoreactivity of the resulting serum was analysed by means of western blot (Fig. 1) in HEK293 cells transfected with plasmids encoding human (Hs) or zebrafish (Dr) ERK2. In the latter, the residue corresponding to serine 284 is a proline (proline 293), so it is not phosphorylatable, and is used as a negative control. It was observed that after stimulation with EGF for 5 min, which induces phosphorylation of canonical TEY residues in both human and zebrafish ERK2s, phosphorylation of ser 284 is detected only in human ERK2.

Once the specificity of the antibody has been demonstrated, the cell sublocalisation of ERK1/2 phosphorylated in ser 301/284 was analysed. To that end, immunofluorescence was performed on HeLa cells, that were fasting (starved) or stimulated with EGF for 5 min (Fig. 2). It was observed, by means of confocal microscopy, that endogenous ERK1/2 phosphorylated in ser 301/284 are located exclusively in the cytoplasm, being completely excluded from the cell nucleus, stained by means of DAPI.

Subsequently, the correlation of the levels of ERK1/2 phosphorylated in ser 301/284 with sensitivity towards BRAF inhibitors in melanoma cells carrying BRAF mutations was analysed. For this purpose, the M249 cell line was used, from which a subline resistant to vemurafenib (vemR) was obtained. The levels of endogenous ERK1/2 phosphorylated in ser 301/284 were analysed by means of western blot (FIG. 3) comparing the resistant subline with the parental line, sensitive to vemurafenib. It was observed that the levels of phospho-ser 301/284 are much higher in the parental line, sensitive to vemurafenib, demonstrating a positive correlation between the levels of phospho-ser 301/284 and the response to the anti-tumour compound.

To verify the previous point, the correlation between the phosphorylation levels of ser 284/301 in ERK1/2 and the sensitivity to vemurafenib in different lines of melanoma was evaluated. It was observed that the reduction in the total activity of ERK1/2, measured by the decrease in the levels of canonical phosphorylation (p-ERK) by means of western blot, after the treatment of the different cell lines with vemurafenib, was correlated with the highest levels of phospho-ser 284/301. It is observed that said levels before treatment with vemurafenib are much higher in the most sensitive lines, 8505C and A375P, compared to those observed in the most resistant lines, MELJUSO and SKMEL2 (FIG. 4). This correlation is also observed when the concentration of vemurafenib necessary to stop the proliferation (GI50) of the different cell lines of melanoma is evaluated (FIG. 5). It is observed that the most sensitive lines to treatment with vemurafenib are 8505C and A375P, which show the highest levels of phospho-ser 284/301 in FIG 4.

## Claims

1. A use of the levels of ERK1 phosphorylated in serine 301 and of the levels of ERK2 phosphorylated in serine 284 as a biomarker to predict *in vitro* the response to treatment with a serine-threonine kinase BRAF inhibitor in a patient.

2. The use according to claim 1, wherein the serine-threonine kinase BRAF inhibitor is vemurafenib and/or dabrafenib.

3. The use according to any of claims 1 or 2, wherein the patient suffers from cancer.

4. The use according to claim 3, wherein the cancer is melanoma or non-small cell lung cancer.

5. The use according to any of claims 1 to 4, wherein the serine-threonine kinase BRAF inhibitor is vemurafenib and the patient suffers from melanoma.

6. The use according to any of claims 1 to 5, wherein the patient is human.

7. An *in vitro* method to predict the response to treatment with a serine-threonine kinase BRAF inhibitor in a patient, wherein said method comprises the following steps:
a. Quantifying the levels of ERK1 phosphorylated in serine 301 and of ERK2 phosphorylated in serine 284 in an isolated biological sample of the patient,
b. Comparing the levels quantified in step (a) with a reference value, wherein said reference value comes from the quantification of the levels of ERK1 phosphorylated in serine 301 and of ERK2 phosphorylated in serine 284 in an isolated biological sample of a patient who does not respond to treatment with a serine-threonine kinase BRAF inhibitor, and
c. Assigning the patient from step (a) to the group of individuals who will respond adequately to treatment when the quantification value obtained in step (a) is significantly higher than the reference value.

8. The method according to claim 7, wherein the serine-threonine kinase BRAF inhibitor is vemurafenib and/or dabrafenib.

9. The method according to any of claims 7 or 8, wherein the patient suffers from cancer.

10. The method according to claim 9, wherein the cancer is melanoma or non-small cell lung cancer.

11. The method according to any of claims 7 to 10, wherein the serine-threonine kinase BRAF inhibitor is vemurafenib and the patient suffers from melanoma.

12. The method according to any of claims 7 to 11, wherein the patient is human.

13. The method according to any of claims 7 to 12, wherein the quantification of the levels of ERK1 phosphorylated in serine 301 and of ERK2 phosphorylated in serine 284 is carried out by using a specific antibody against the peptide consisting of SEQ ID NO: 1.

14. A specific antibody against the peptide consisting of SEQ ID NO: 1.

15. A kit comprising the antibody according to claim 14.

16. A use of the antibody according to claim 14 or of the kit according to claim 15 to predict *in vitro* the response to treatment with a serine-threonine kinase BRAF inhibitor in a patient.

17. The use according to claim 16, wherein the serine-threonine kinase BRAF inhibitor is vemurafenib and/or dabrafenib.

18. The use according to any of claims 16 or 17, wherein the patient suffers from cancer.

19. The use according to claim 18, wherein the cancer is melanoma or non-small cell lung cancer.

20. The use according to any of claims 16 to 19, wherein the serine-threonine kinase BRAF inhibitor is vemurafenib and the patient suffers from melanoma.

21. The use according to any of claims 16 to 20, wherein the patient is human.
